# EUROPEAN PATENT SPECIFICATION

(11) **EP 4 658 252 B1**
(45) Date of publication and mention of the grant of the patent: **09.12.2026**
(21) Application number: 24701842.7
(22) Date of filing: 22.01.2024
(51) Int. Cl.: A61K 9/00, A61K 9/46, A61K 9/48, A61K 47/02, A61K 47/12, A61K 47/24, A61K 47/26, A61K 31/00, A24B 13/00, A24B 15/167, A24B 15/28

(54) **ORAL RECEPTACLE**
MUNDBEHÄLTNIS
RÉCEPTACLE BUCCAL

(43) Date of publication of application: 10.12.2025
(60) Divisional application: 26158298.5 / 4 785 933
(73) Proprietor: Splash tm GmbH, 49733 Haren (DE)
(72) Inventor: HAGEN, Klaus, 49733 Haren (DE)
(74) Representative: Cyrson, Matthew Dominic
(86) International application number: PCT/EP2024/051438
(87) International publication number: WO 2025/157386

(56) References cited:
- AU-A1- 2021 289 162
- US-A- 4 036 228
- US-A1- 2009 297 569

## Description

The present invention relates to an oral receptacle.

Consuming or inhaling some medicinal or psychoactive compounds such as nicotine, cannabidiol (CBD), or tetrahydrocannabinol (THC) may conventionally rely on burning substances, for example burning tobacco in cigarettes, and inhaling the smoke produced.

Electronic cigarettes (e-cigarettes) are an alternative to this, in which electrical energy is used to produce a vapour to be inhaled, instead of smoke.

The inhalation of the smoke or vapour may have medicinal effects on the user, or psychoactive effects such as stimulatory or relaxing effects. The effects produced are due to the chemicals contained within the cigarette or e-cigarette, for example nicotine, CBD or THC.

As well as the stimulating effects from the chemicals, the smoke or vapour can provide a physical sensation in the user's mouth.

There are well known health concerns regarding smoke inhalation from cigarettes, including lung and throat cancer. People often seek alternative ways to consume the chemicals which are less hazardous than inhaling smoke.

US 4,036,228 A discloses an osmotic device that uses a gas generating means such as an effervescent couple to deliver an active agent at a controlled and continuous rate over a prolonged period of time from the device to an environment of use.

AU2021289162 discloses a pouch comprising a receptacle body. In the receptacle body is nicotine and an effervescent couple comprising citric acid and sodium carbonate.

Oral nicotine pouches and tobacco pouches, such as snus, are a common alterative, the contents of which are absorbed through the oral mucosa. However, prolonged use of these pouches can result in the risk of oral injury from repeated placement of the said pouches in the same place.

Whilst e-cigarettes are considered to pose fewer health concerns, the requirement to provide an electrical energy supply can be inconvenient, requiring frequent recharging or replacement of batteries or devices, along with associated environmental concerns. The user is also often not able to accurately gauge how much nicotine they have consumed per day due to a given quantity of liquid being used to produce vapour over a long duration.

It would therefore be desirable to provide a way of delivering nicotine accompanied by a mouth sensation, without conventional associated production of smoke or vapour.

It is an object of the present invention to reduce or substantially obviate the above problems.

According to a first aspect of the invention, there is provided an oral receptacle comprising: a receptacle body for being received in a user's mouth, a nicotine component for producing a psychoactive effect on a user, the nicotine component being located within the receptacle body, and an effervescent means comprising an acid and a carbonate located within the receptacle body for forming a foam in the user's mouth which distributes the nicotine component, the effervescent means further comprising a liquid receptacle containing liquid.

Harmful inhalation of smoke is avoided, and the user is still able to experience a medicinal and/or psychoactive effect. The receptacle body can be placed in the user's mouth to release the carbonate and acid within, which can then mix with and/or dissolve in the user's saliva to react to produce a foam via generation of carbon dioxide. The foam covers a large surface area in the user's mouth and thus increases the rate of absorption of the medicinal and/or psychoactive component by the oral mucosa of the user's mouth. This is known to be the fastest route to the brain of an active ingredient and thus the medicinal and/or psychoactive effect is achieved quickly after the foam is absorbed. The foam also allows for an enhanced physical mouth experience.

The liquid receptacle provides additional liquid, in case the user's saliva is insufficient to fully mix and/or dissolve the acid and carbonate and/or the medicinal and/or psychoactive components.

Advantageously, the liquid may contain a stabiliser, for example polysorbate 20 and/or lecithin, for stabilising the foam. The stabiliser maintains the structure of the foam in the user's mouth and so may provide a longer lasting mouth sensation.

Optionally, the liquid receptacle is flexible. The user can apply pressure to the liquid receptacle to cause it to burst and release the liquid within.

Preferably, the receptacle body and/or the liquid receptacle is frangible. Following breakage of the liquid receptacle, the liquid can be released into its surroundings to dissolve the acid and carbonate and/or medicinal and/or psychoactive component to form the foam.

Beneficially, the liquid receptacle may comprise at least one liquid-transfer conduit between the liquid receptacle and the acid and the carbonate and/or the nicotine component. The release of liquid, upon perforation of the liquid receptacle, is directed into the acid and the carbonate to more effectively produce the foam.

Optionally, the liquid-transfer conduit has a seal which is frangible when pressure is applied to the liquid receptacle. The frangible seal is an area of weakness in the liquid receptacle and promotes perforation at the seal when pressure is applied. This directs the liquid to the acid and the carbonate to more efficiently produce the foam. Preferably, the receptacle body comprises plant fibres. The plant fibres may be bound by a resin. The plant fibres, and/or resin where present, may be food safe and/or digestible, so that the entire receptacle may be consumed leaving no waste product to discard.

Optionally, the acid and the carbonate are powders. This provides a higher surface area for the liquid and/or saliva to efficiently produce the foam. The powders may be in compressed form, such as a tablet. Whilst a powder is preferred, it will be appreciated that other solid forms may be considered. Alternatively, one or both of the acid and carbonate may be provided in liquid form.

Beneficially, the acid may comprise citric acid and/or the carbonate comprises sodium carbonate. The citric acid and sodium carbonate are food-safe and easily accessible compounds. The reaction of the acid and the carbonate, preferably via liquid, causes the production of carbon dioxide which generates the foam.

Preferably, the receptacle body is soluble in water. This allows for the receptacle body to dissolve when placed in the mouth due to the presence of saliva. This causes the release of the substances within the receptacle body without necessarily requiring pressure to be applied to the oral receptacle.

Optionally, the receptacle body is insoluble in water. This gives the user more control on when to activate the receptacle in the mouth. The user can apply pressure to perforate the receptacle to release the substances within the receptacle body.

Preferably, the receptacle body comprises at least one fin. The fins allow the user to easily move and manipulate the receptacle within their mouth to position the receptacle correctly.

Advantageously, the effervescent means and/or the nicotine component may be defined by a plurality of spaced apart components, preferably at least ten thereof, which are distributed within the receptacle body. Having a larger number of components provides a higher surface area for the effervescent means and the nicotine component to mix such that the nicotine component are distributed evenly throughout the foam produced. The components may be capsules, balls or beads of the effervescent means and/or the nicotine component.

Preferably, the oral receptacle is a tobacco industry product. The oral receptacle is therefore preferably, but not exclusively, a non-smoke substitute for conventional tobacco products such as cigarettes, and e-cigarettes. The oral receptacle is also a substitute for nicotine pouches and tobacco pouches; removing the risk of the user having oral injuries.

The invention will now be more particularly described, by way of example only, with reference to the accompanying drawings, in which:
Figure 1 is a perspective view of a first embodiment of an oral receptacle in accordance with the first aspect of the invention containing a liquid receptacle;
Figure 2 is a front view of a second embodiment of the oral receptacle in accordance with the first aspect of the invention containing the liquid receptacle;
Figure 3 is a perspective view of the second embodiment of the oral receptacle of Figure 2;
Figure 4 is a side view of the second embodiment of the oral receptacle of Figure 2;
Figure 5 is a perspective view of a third embodiment of the oral receptacle which is not according to the invention without a liquid receptacle;
Figure 6 is a perspective view of a fourth embodiment of the oral receptacle which is not according to the invention showing a plurality of spaced apart components; and
Figure 7 is a front view of a fifth embodiment of the oral receptacle in accordance with the first aspect of the invention.

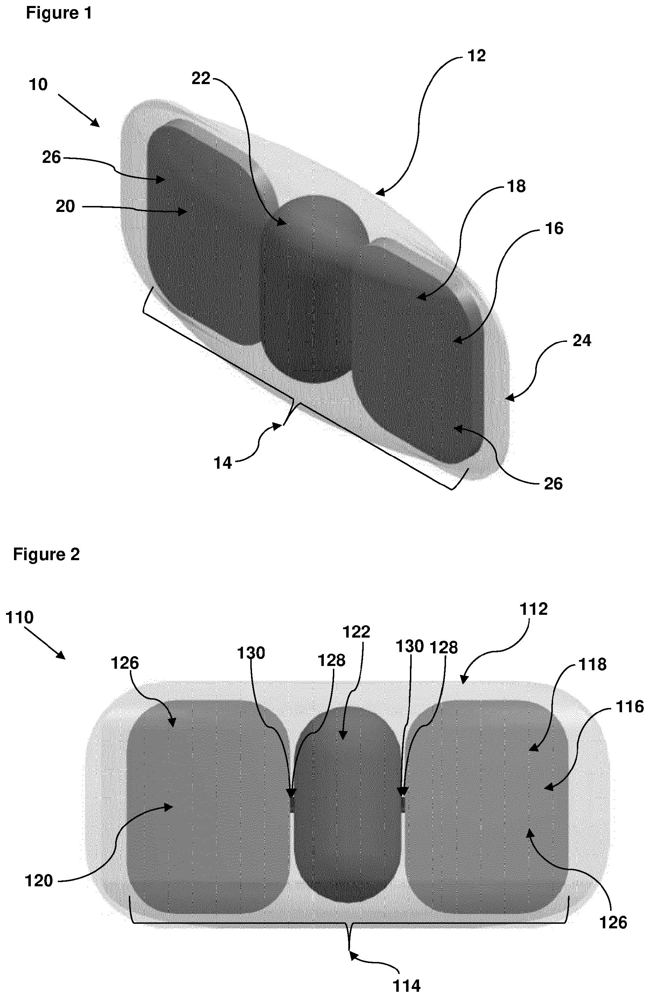

Referring to Figure 1, there is shown a first embodiment of an oral receptacle 10 having a receptacle body 12, an effervescent means 14 comprising an acid 16 and a carbonate 18, and a medicinal and/or psychoactive component 20. The oral receptacle 10 is a tobacco industry product.

The receptacle body 12 is a case or pouch. In Figure 1, the case is illustrated as being transparent to allow the effervescent means 14 and medicinal and/or psychoactive component 20 to be seen within. However, the case may not necessarily be transparent, and may be opaque or translucent.

The receptacle body 12 is also insoluble in water and perforable so that when the user applies pressure to the receptacle body 12, the components within the receptacle body 12 are released. The receptacle body 12 also comprises plant fibres, for example hemp or cellulose. The plant fibres are preferably edible and/or digestible. It can be appreciated that the receptacle body 12 may be soluble in water and perforable.

The receptacle body 12 is flexible for ease of manipulation in the user's mouth so that the user can fit the receptacle body 12 between their gums or teeth. Additionally, the flexibility allows pressure to be applied by the user through the receptacle body 12 to a liquid receptacle 22 within to burst both the receptacle body 12 and the liquid receptacle 22. The liquid receptacle 22 containing liquid may also form part of the effervescent means.

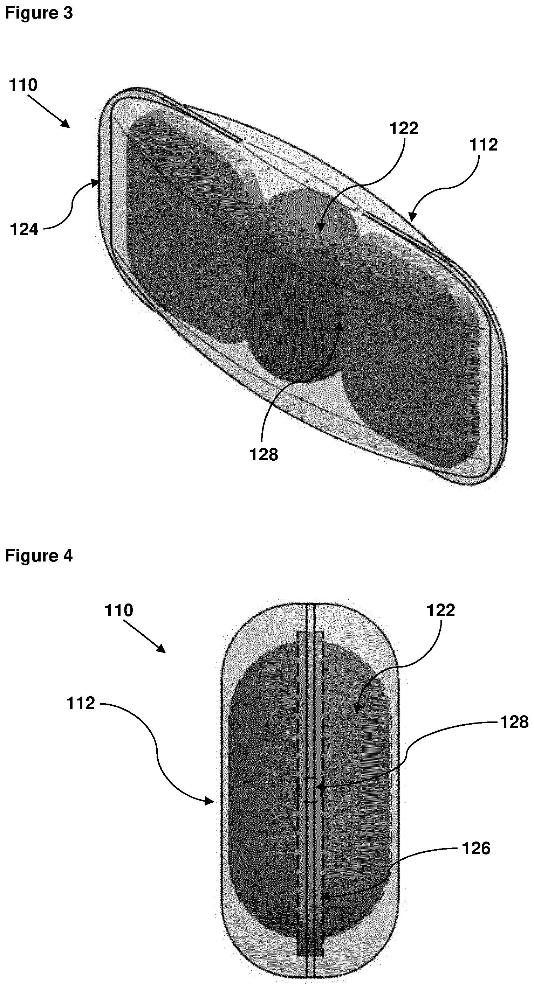

The receptacle body 12 comprises at least one fin 24 as shown more clearly in Figure 3. The fin 24 is located substantially around a perimeter of the receptacle body 12 and extends outwards from the receptacle body 12. The fin 24 provides the user with a surface to more easily move the oral receptacle 10 around their mouth.

The medicinal and/or psychoactive component 20 comprises nicotine and may have one or more of caffeine, cannabidiol, tetrahydrocannabinol, a vitamin, a pharmaceutical and/or alcohol. The medicinal and/or psychoactive component 20 is in the form of a powder, although in an alternative embodiment the medicinal and/or psychoactive component 20 may be in liquid form. The medicinal and/or psychoactive component 20 provides relief, stimulation or other medicinal and/or psychoactive effect on the user upon absorption. The medicinal and/or psychoactive component 20 is located within the receptacle body 12.

The effervescent means 14 is located within the receptacle body 12. The acid 16 and the carbonate 18 are powders and in this embodiment are citric acid and sodium carbonate. The acid 16 and the carbonate 18 and the medicinal and/or psychoactive component 20 may be in the form of a tablet 26 or pill. The carbonate 18, acid 16, medicinal and/or psychoactive component 20 may be intermixed in the or each tablet 26 or pill. There are shown two such tablets 26 in Figure 1 for provision and distribution of a larger amount of the medicinal and/or psychoactive component 20.

The liquid receptacle 22 is located within the receptacle body 12 and disposed between the tablets 26. The liquid receptacle 22 is a further component of the effervescent means 14, and contains liquid. The liquid is preferably water or any other edible liquid. The liquid receptacle 22 is flexible and also perforable such that the liquid can be released to mix with the acid 16 and carbonate 18 to form a foam. The liquid receptacle 22 is a pill case containing liquid and may be ovoid in shape.

The liquid comprises a stabiliser, for example polysorbate 20 - also known as Tween (RTM) 20 - or lecithin. The stabiliser stabilises the foam produced from the mixing of the liquid and the acid 16 and carbonate 18, and maintains the structure of the foam until the user ingests it.

In use, the user inserts the oral receptacle 10 into their mouth and bites down on the receptacle body 12. The receptacle body 12 is configured so that when pressure is applied by the user's teeth or gums, the pressure is also applied to the liquid receptacle 22 which causes the receptacle body 12 and the liquid receptacle 22 to burst, releasing the liquid.

The released liquid mixes with and dissolves the acid 16 and carbonate 18 to form the foam. The foam combines with the medicinal and/or psychoactive components 20 to form a medicinal and/or psychoactive foam which is distributed throughout the user's mouth. The foam has a large surface area in the user's mouth, and therefore the active ingredient of the medicinal and/or psychoactive component 20 may be easily absorbed by the user through the oral mucosa.

Referring to Figures 2 to 4, there is shown a second embodiment of the oral receptacle 110. Similar features to the first embodiment have similar reference numerals with 100 added. Further detailed description has been omitted for brevity.

The oral receptacle 110 comprises the receptacle body 112, the effervescent means 114 comprising the acid 116, the carbonate 118, and the liquid receptacle 122, and the medicinal and/or psychoactive component 120.

The fins 124 are shown more clearly in Figure 3 outlines by the solid black lines.

Two liquid-transfer conduits 128 extend from the liquid receptacle 122 to the tablets 126 comprising the acid 116 and the carbonate 118 and/or the medicinal and/or psychoactive component 120. The liquid-transfer conduits 128 are best seen in Figure 2, and may be termed tubes.

The liquid-transfer conduits 128 may each have a seal 130 which is frangible when pressure is applied to the liquid receptacle 122. The seal 130 may be located at the interface between the liquid-transfer conduit 128 and the tablet 126. The seal 130 provides a weakness, or predetermined breaking point, at which the liquid receptacle 122 breaks or perforates and allows for the liquid to be directed into the tablets 126. This arrangement efficiently produces the medicinal and/or psychoactive foam as the liquid is focussed directly onto the tablets 126 and thus all of the liquid dissolves the acid 116 and carbonate 118 and medicinal and/or psychoactive components 120. Although the term seal is used, it will be appreciated that this may in fact only be a portion of thinner wall of the liquid receptacle 122, or an otherwise weakened portion, which therefore allows this area to break in preference of the remainder of the liquid receptacle.

Figure 4 shows that a thickness of the liquid receptacle 122 is thicker than a thickness of the tablet 126. The location of the liquid-transfer conduit 128 is located in a central portion of the liquid receptacle 122, the position of the liquid-transfer conduit 128 shown by a dashed line. It is also shown that a cross section of the receptacle body 112 and a cross section of the liquid receptacle 122 is substantially elliptical.

The mechanism of use is the same as the first embodiment, except that the liquid is directly delivered to the tablets 126 via the liquid-transfer conduits 128 upon perforation of the receptacle body 112 and liquid receptacle 122.

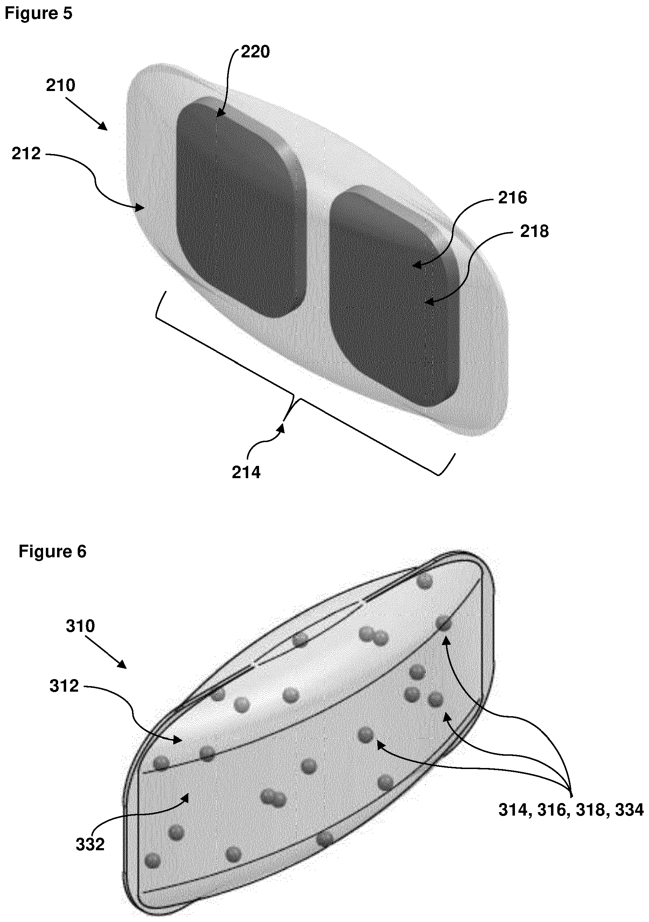

Referring to Figure 5, there is shown a third embodiment of the oral receptacle 210. Similar features to the first embodiment have similar reference numerals with 200 added. Further detailed description has been omitted for brevity.

The liquid receptacle is omitted in the third embodiment. The oral receptacle 210 comprises the receptacle body 212, the effervescent means 214 comprising the acid 216 and the carbonate 218, and the medicinal and/or psychoactive component 220.

The effervescent means 214 in this embodiment only comprises the acid 216 and the carbonate 218.

In use, the user inserts the oral receptacle 210 into their mouth and bites down on the receptacle body 212. The receptacle body 212 is configured so that when pressure is applied by the user's teeth or gums, the receptacle body 212 bursts.

The exposed acid 216 and carbonate 218 dissolve and mix with the user's saliva to form the foam. The foam combines with the medicinal and/or psychoactive components 220 to form a medicinal and/or psychoactive foam which is distributed throughout the user's mouth. The medicinal and/or psychoactive component 220 is easily absorbed by the user.

Referring to Figure 6, there is shown a fourth embodiment of the oral receptacle 310. Similar features to the first embodiment have similar reference numerals with 300 added. Further detailed description has been omitted for brevity.

The liquid receptacle is omitted in the fourth embodiment. The oral receptacle 310 comprises the receptacle body 312, the effervescent means 314 comprising the acid 316 and the carbonate 318 and the medicinal and/or psychoactive component 320.

The receptacle body 312 comprises a matrix 332 in which the effervescent means 314 and medicinal and/or psychoactive component 320 are distributed or suspended. The matrix 332 comprises the same material as the receptacle body 312.

The effervescent means 314 and the medicinal and/or psychoactive component 320 are defined by a plurality of spaced apart components 334 which are distributed within the matrix 332 of the receptacle body 312. There are shown twenty-two components 334 in Figure 6, and thus there are more than ten components 334 within the receptacle body 12. Each of the plurality of components 334 has a smaller surface area compared to the surface area of the tablets 26 of the previous embodiments.

In use, the user inserts the oral receptacle 310 into their mouth. The receptacle body 312 is soluble in water in this embodiment and thus dissolves in the user's mouth when in contact with saliva. It can be appreciated that the receptacle body 312 can be insoluble in water. Pressure may be applied by the user to speed up the process.

Once the receptacle body 312 is dissolved, the exposed acid 316 and carbonate 318 dissolve and mix with the user's saliva to form the foam. The foam combines with the medicinal and/or psychoactive components 320 to form a medicinal and/or psychoactive foam which is distributed throughout the user's mouth. The foam, and therefore the medicinal and/or psychoactive component 320 is easily absorbed by the user.

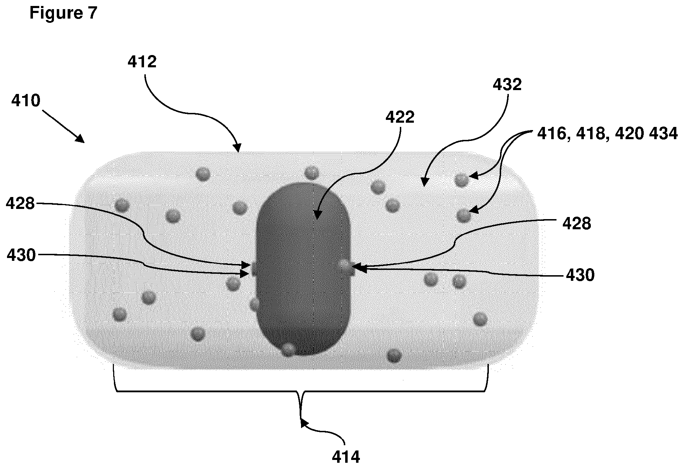

Referring to Figure 7, there is shown a fifth embodiment of the oral receptacle 410. Similar features to the first embodiment have similar reference numerals with 400 added. Further detailed description has been omitted for brevity.

The fifth embodiment is similar to the fourth embodiment with the addition of a liquid receptacle 422. The effervescent means 414 comprises the liquid receptacle 422 together with a plurality of components 434 containing acid 416 and carbonate 418. The plurality of components 434 may also contain the medicinal and/or psychoactive components 420.

The liquid receptacle 422 may include a liquid-transfer conduit 428 or protrusion, which is preferably tubular. The liquid-transfer conduit 428 or protrusion may extend from the liquid receptacle 422 into the matrix 432 and may direct liquid into the matrix 432.

The liquid receptacle 422 may include at least one seal 430, or predetermined breaking point, which is frangible to allow emission of liquid when the liquid inside the liquid receptacle is pressurised. The liquid may be pressurised via compression or deformation of the liquid receptacle 422.

The receptacle body 412 is insoluble in water in this embodiment, and the liquid may be dispensed therefrom only once the receptacle body 412 is bitten through. However, it is envisaged that the receptacle body 412 could be soluble.

In use, the user inserts the oral receptacle 410 into their mouth and bites down on the receptacle body 412. The oral receptacle 410 may be punctured or broken by this biting.

When the receptacle body 412 is bitten through and/or deformed by the user's teeth or gums, pressure is applied to the liquid receptacle 422 which causes the receptacle body 412 and the liquid receptacle 422 to burst, preferably at the pre-determined breaking points, releasing the liquid.

The liquid is dispersed from the burst liquid receptacle 422 to the plurality of components 434 via the liquid-transfer conduits 428.

The released liquid mixes with and dissolves the acid 416 and carbonate 418 to react to form carbon dioxide which forms the foam. The foam includes the medicinal and/or psychoactive components 420 forming a medicinal and/or psychoactive foam which is distributed throughout the user's mouth. The foam, and therefore the medicinal and/or psychoactive component 420 is easily absorbed by the user.

In any of the aforementioned embodiments, the receptacle body may have a width of between 30 mm and 35 mm, for example 32 mm and 35 mm, a height of 15 mm and a thickness of 8 mm. However, it is appreciated that the receptacle body may have any other combination of dimensions suitable to fit within a user's mouth.

The liquid receptacle, if present, may have a diameter of 6.5 mm and a height of 12 mm. It is appreciated that the liquid receptacle may have any other combination of dimensions suitable to fit within the receptacle body.

The or each tablet, if present, may have a height of 13 mm and a width of 10 mm, although other dimensions may be considered to fit within the receptacle body.

In an alternative embodiment, the receptacle body comprises gelatine.

In an alternative embodiment, the acid comprises acetic acid, tartaric acid, malic acid, folic acid, fumaric acid, lactic acid or other edible acid.

In an alternative embodiment, the carbonate may comprise any other edible carbonate compound, such as calcium carbonate or sodium bicarbonate.

In an alternative embodiment, there may be one or more than two tablets present in the receptacle body.

In an alternative embodiment, the acid and carbonate and the medicinal and/or psychoactive component may be spaced apart from one another in different tablets.

In an alternative embodiment, the tablet may be a receptacle containing the acid and carbonate and/or the medicinal and/or psychoactive component in solid form. The receptacle may be a flexible pill or receptacle similar to the receptacle body and liquid receptacle such that pressure can be applied to burst the receptacle and release the acid and carbonate and/or the medicinal and/or psychoactive component to be dissolved in liquid and/or saliva.

In an alternative embodiment, the acid and the carbonate and the medicinal and/or psychoactive component may be in solution.

In an alternative embodiment, the liquid may comprise at least one of the acid and the carbonate and the medicinal and/or psychoactive component.

In an alternative embodiment, the medicinal and/or psychoactive component may additionally include any other medicinal and/or psychoactive substance, for example paracetamol or ibuprofen.

It is therefore possible to provide a means for consuming medicinal or psychoactive compounds without inhalation of harmful smoke, as well as distributing the compounds within the user's mouth whilst providing a pleasant mouth sensation.

The words 'comprises/comprising' and the words 'having/including' when used herein with reference to the present invention are used to specify the presence of stated features, integers, steps, or components, but do not preclude the presence or addition of one or more other features, integers, steps, components, or groups thereof.

It is appreciated that certain features of the invention, which are, for clarity, described in the context of separate embodiments, may also be provided in combination in a single embodiment. Conversely, various features of the invention which are, for brevity, described in the context of a single embodiment, may also be provided separately or in any suitable sub-combination.

The embodiments described above are provided by way of example only, and various changes and modifications will be apparent to persons skilled in the art without departing from the scope of the present invention as defined by the appended claims.

## Claims

1. An oral receptacle (10, 110, 210, 310, 410) comprising:
a receptacle body (12, 112, 212, 312, 410) for being received in a user's mouth,
a nicotine component (20, 120, 220, 320, 420) for producing a psychoactive effect on a user, the nicotine component (20, 120, 220, 320, 420) being located within the receptacle body (12, 112, 212, 312, 412), and
an effervescent means (14, 114, 214, 314, 414) comprising an acid (16, 116, 216, 316, 416) and a carbonate (18, 118, 218, 318, 418) located within the receptacle body (12,112, 212, 312, 412) for forming a foam in the user's mouth which distributes the nicotine component (20, 120, 220, 320, 420),
the effervescent means (14, 114, 414) further comprising a liquid receptacle (22, 122, 422) containing liquid.

2. An oral receptacle (10, 110, 410) as claimed in claim 1, wherein the liquid contains a stabiliser, for example polysorbate 20 or lecithin, for stabilising the foam.

3. An oral receptacle (10, 110, 410) as claimed in any one of the preceding claims, wherein the receptacle body (12, 112, 212, 312, 412) is flexible for ease of manipulation in a mouth of the user and/or the liquid receptacle (22, 122, 422) is flexible.

4. An oral receptacle (10, 110, 410) as claimed in any one of the preceding claims wherein the receptacle body (12, 112, 212, 312, 412) and/or the liquid receptacle (22, 122, 422) is frangible.

5. An oral receptacle (110, 410) as claimed in any one of the preceding claims, wherein the liquid receptacle (122, 422) comprises at least one liquid-transfer conduit (128, 428) between the liquid receptacle (122, 422) and the acid (116, 416) and the carbonate (118, 418) and/or the nicotine component (120, 420).

6. An oral receptacle (110, 410) as claimed in claim 5, wherein the liquid-transfer conduit (128. 428) has a seal (130, 430) which is frangible when pressure is applied to the liquid receptacle (122, 422).

7. An oral receptacle (10, 110, 210, 310, 410) as claimed in any one of the preceding claims, wherein the receptacle body (12, 112, 212, 312, 412) comprises plant fibres.

8. An oral receptacle (10, 110, 210, 310, 410) as claimed in any one of the preceding claims, wherein the acid (16, 116, 216, 316, 416) and the carbonate (18, 118, 218, 318, 418) are powders.

9. An oral receptacle (10, 110, 210, 310, 410) as claimed in any one of the preceding claims, wherein the acid (16, 116, 216, 316, 416) comprises citric acid and/or the carbonate (18, 118, 218, 318, 418) comprises sodium carbonate.

10. An oral receptacle (10, 110, 210, 310, 410) as claimed in any one of the preceding claims, wherein the receptacle body (12, 112, 212, 312, 412) is soluble in water.

11. An oral receptacle (10, 110, 210, 410) as claimed in any one of claims 1 to 9, wherein the receptacle body (12, 112, 212, 412) is insoluble in water.

12. An oral receptacle (10, 110, 210, 310, 410) as claimed in any one of the preceding claims, wherein the receptacle body (12, 112, 212, 312, 412) comprises at least one fin (24, 124).

13. An oral receptacle (310, 410) as claimed in any one of the preceding claims, wherein the effervescent means (314, 414) and/or the nicotine component (320, 420) are defined by a plurality of spaced apart components (334, 434), preferably at least ten thereof, which are distributed within the receptacle body (312, 412).

14. An oral receptacle (10, 110, 210, 310, 410) as claimed in any one of the preceding claims, wherein the oral receptacle (10, 110, 210, 310, 410) is a tobacco industry product.

## Patentansprüche

1. Mundbehältnis (10, 110, 210, 310, 410), umfassend:
einen Behältniskörper (12, 112, 212, 312, 412) zur Aufnahme in einem Mund eines Benutzers,
eine Nikotinkomponente (20, 120, 220, 320, 420) zum Hervorrufen einer psychoaktiven Wirkung bei einem Benutzer, wobei sich die Nikotinkomponente (20, 120, 220, 320, 420) innerhalb des Behältniskörpers (12, 112, 212, 312, 412) befindet, und
ein aufschäumendes Mittel (14, 114, 214, 314, 414), das eine Säure (16, 116, 216, 316, 416) und ein Carbonat (18, 118, 218, 318, 418) umfasst, das sich innerhalb des Behältniskörpers (12, 112, 212, 312, 412) befindet, zum Bilden eines Schaums in dem Mund des Benutzers, der die Nikotinkomponente (20, 120, 220, 320, 420) verteilt, wobei das aufschäumende Mittel (14, 114, 414) ferner ein Flüssigkeitsbehältnis (22, 122, 422) umfasst, das eine Flüssigkeit enthält.

2. Mundbehältnis (10, 110, 410) nach Anspruch 1, wobei die Flüssigkeit einen Stabilisator, beispielsweise Polysorbat 20 oder Lecithin, zum Stabilisieren des Schaums enthält.

3. Mundbehältnis (10, 110, 410) nach einem der vorhergehenden Ansprüche, wobei der Behältniskörper (12, 112, 212, 312, 412) zur einfacheren Manipulation in einem Mund des Benutzers flexibel ist und/oder das Flüssigkeitsbehältnis (22, 122, 422) flexibel ist.

4. Mundbehältnis (10, 110, 410) nach einem der vorhergehenden Ansprüche, wobei der Behältniskörper (12, 112, 212, 312, 412) und/oder das Flüssigkeitsbehältnis (22, 122, 422) zerbrechlich sind.

5. Mundbehältnis (110, 410) nach einem der vorhergehenden Ansprüche, wobei das Flüssigkeitsbehältnis (122, 422) mindestens eine Flüssigkeitsübertragungsleitung (128, 428) zwischen dem Flüssigkeitsbehältnis (122, 422) und der Säure (116, 416) und dem Carbonat (118, 418) und/oder der Nikotinkomponente (120, 420) umfasst.

6. Mundbehältnis (110, 410) nach Anspruch 5, wobei die Flüssigkeitsübertragungsleitung (128, 428) eine Dichtung (130, 430) aufweist, die zerbrechlich ist, wenn Druck auf das Flüssigkeitsbehältnis (122, 422) ausgeübt wird.

7. Mundbehältnis (10, 110, 210, 310, 410) nach einem der vorhergehenden Ansprüche, wobei der Behältniskörper (12, 112, 212, 312, 412) Pflanzenfasern umfasst.

8. Mundbehältnis (10, 110, 210, 310, 410) nach einem der vorhergehenden Ansprüche, wobei die Säure (16, 116, 216, 316, 416) und das Carbonat (18, 118, 218, 318, 418) Pulver sind.

9. Mundbehältnis (10, 110, 210, 310, 410) nach einem der vorhergehenden Ansprüche, wobei die Säure (16, 116, 216, 316, 416) Citronensäure umfasst und/oder das Carbonat (18, 118, 218, 318, 418) Natriumcarbonat umfasst.

10. Mundbehältnis (10, 110, 210, 310, 410) nach einem der vorhergehenden Ansprüche, wobei der Behältniskörper (12, 112, 212, 312, 412) in Wasser löslich ist.

11. Mundbehältnis (10, 110, 210, 410) nach einem der Ansprüche 1 bis 9, wobei der Behältniskörper (12, 112, 212, 412) in Wasser unlöslich ist.

12. Mundbehältnis (10, 110, 210, 310, 410) nach einem der vorhergehenden Ansprüche, wobei der Behältniskörper (12, 112, 212, 312, 412) mindestens eine Rippe (24, 124) umfasst.

13. Mundbehältnis (310, 410) nach einem der vorhergehenden Ansprüche, wobei das aufschäumende Mittel (314, 414) und/oder die Nikotinkomponente (320, 420) durch eine Vielzahl von beabstandeten Komponenten (334, 434) definiert sind, vorzugsweise mindestens zehn davon, die innerhalb des Behältniskörpers (312, 412) verteilt sind.

14. Mundbehältnis (10, 110, 210, 310, 410) nach einem der vorhergehenden Ansprüche, wobei das Mundbehältnis (10, 110, 210, 310, 410) ein Produkt der Tabakindustrie ist.

## Revendications

1. Un réceptacle oral (10, 110, 210, 310, 410) comprenant :
un corps de réceptacle (12, 112, 212, 312, 412) destiné à être placé dans la bouche
d'un utilisateur,
un composant à base de nicotine (20, 120, 220, 320, 420) destiné à produire un effet psychoactif chez un utilisateur, le composant à base de nicotine (20, 120, 220, 320, 420) étant situé à l'intérieur du corps de réceptacle (12, 112, 212, 312, 412), et
des moyens effervescents (14, 114, 214, 314, 414) comprenant un acide (16, 116, 216, 316, 416) et un carbonate (18, 118, 218, 318, 418), situés à l'intérieur du corps de réceptacle (12, 112, 212, 312, 412), pour former une mousse dans la bouche de l'utilisateur, qui distribue le composant à base de nicotine (20, 120, 220, 320, 420),
les moyens effervescents (14, 114, 414) comprenant en outre un réceptacle de liquide (22, 122, 422) contenant un liquide.

2. Un réceptacle oral (10, 110, 410) selon la revendication 1, dans lequel le liquide contient un stabilisant, par exemple du polysorbate 20 ou de la lécithine, destiné à stabiliser la mousse.

3. Un réceptacle oral (10, 110, 410) selon l'une quelconque des revendications précédentes,
dans lequel le corps de réceptacle (12, 112, 212, 312, 412) est flexible pour faciliter la manipulation dans la bouche de l'utilisateur et/ou le réceptacle de liquide (22, 122, 422) est flexible.

4. Un réceptacle oral (10, 110, 410) selon l'une quelconque des revendications précédentes,
dans lequel le corps de réceptacle (12, 112, 212, 312, 412) et/ou le réceptacle de liquide
(22, 122, 422) est frangible.

5. Un réceptacle oral (110, 410) selon l'une quelconque des revendications précédentes, dans lequel le réceptacle de liquide (122, 422) comprend au moins un conduit de transfert de liquide (128, 428) entre le réceptacle de liquide (122, 422) et l'acide (116, 416) et le carbonate (118, 418) et/ou au composant à base de nicotine (120, 420).

6. Un réceptacle oral (110, 410) selon la revendication 5, dans lequel le conduit de transfert de liquide (128, 428) comporte un joint d'étanchéité (130, 430) qui est frangible sous l'effet d'une pression
appliquée au réceptacle de liquide (122, 422).

7. Un réceptacle oral (10, 110, 210, 310, 410) selon l'une quelconque des revendications précédentes,
dans lequel le corps de réceptacle (12, 112, 212, 312, 412) comprend des fibres végétales.

8. Un réceptacle oral (10, 110, 210, 310, 410) selon l'une quelconque des revendications précédentes,
dans lequel l'acide (16, 116, 216, 316, 416) et le carbonate (18, 118, 218, 318, 418) se présentent sous forme de poudres.

9. Un réceptacle oral (10, 110, 210, 310, 410) selon l'une quelconque des revendications précédentes,
dans lequel l'acide (16, 116, 216, 316, 416) comprend de l'acide citrique et/ou le carbonate (18, 118, 218, 318, 418) comprend du carbonate de sodium.

10. Un réceptacle oral (10, 110, 210, 310, 410) selon l'une quelconque des revendications précédentes,
dans lequel le corps de réceptacle (12, 112, 212, 312, 412) est soluble dans l'eau.

11. Un réceptacle oral (10, 110, 210, 410) selon l'une quelconque des revendications 1 à 9,
dans lequel le corps de réceptacle (12, 112, 212, 412) est insoluble dans l'eau.

12. Un réceptacle oral (10, 110, 210, 310, 410) selon l'une quelconque des revendications précédentes,
dans lequel le corps de réceptacle (12, 112, 212, 312, 412) comprend au moins une ailette (24, 124).

13. Un réceptacle oral (310, 410) selon l'une quelconque des revendications précédentes, dans lequel les moyens effervescents (314, 414) et/ou le composant à base de nicotine (320, 420) sont définis par une pluralité de composants (334, 434) espacés les uns des autres, de préférence au moins dix, répartis à l'intérieur du corps de réceptacle (312, 412).

14. Un réceptacle oral (10, 110, 210, 310, 410) selon l'une quelconque des revendications précédentes,
dans lequel ledit réceptacle oral (10, 110, 210, 310, 410) est un produit de l'industrie du tabac.
